# FASCICULE DE BREVET EUROPEEN

(11) **EP 1 404 845 B1**
(45) Date de publication et mention de la délivrance du brevet: **15.12.2004**
(21) Numéro de dépôt: 02767549.5
(22) Date de dépôt: 04.07.2002
(51) Int. Cl.: C12N 15/65, C12N 5/10, C12N 5/08, C12Q 1/02, C12Q 1/68, A61K 48/00

(54) **UTILISATION DU GENE MARQUEUR CD24 POUR LA SELECTION DE KERATINOCYTES SOUCHES TRANSFORMES PAR DES SEQUENCES EXOGENES**
VERWENDUNG DES CD24-MARKERGENS ZUR SELEKTION VON KERATINOZYTEN DIE MIT EINEM EXOGENEN SEKVENS TRANSFORMIERT SIND
USE OF THE CD24 MARKER GENE FOR THE SELECTION OF KERATINOCYTE STEM CELLS TRANSFORMED BY EXOGENOUS SEQUENCES

(30) Priorité: 06.07.2001 FR 0108984
(43) Date de publication de la demande: 07.04.2004
(73) Titulaire: Centre National de la Recherche Scientifique, 75016 Paris (FR)
(72) Inventeur: MAGNALDO, Thierry, F-75018 Paris (FR); SARASIN, Alain, F-94320 Thiais (FR)
(74) Mandataire: Grosset-Fournier, Chantal Catherine
(86) Numéro de dépôt international: PCT/FR2002/002347
(87) Numéro de publication internationale: WO 2003/004655

(56) Documents cités:
- US-A- 5 804 177
- MAGNALDO T ET AL: "CD24 (HEAT STABLE ANTIGEN, NECTADRIN), A NOVEL KERATINOCYTE DIFFERENTIATION MARKER, IS PREFERENTIALLY EXPRESSED IN AREAS OF THEHAIR FOLLICLE CONTAINING THE COLONY-FORMING CELLS" JOURNAL OF CELL SCIENCE, ESSEX, GB, vol. 109, no. 13, 1996, pages 3035-3045, XP008001588
- REDONDO P ET AL: "CD24 EXPRESSION ON HUMAN KERATINOCYTES" EXPERIMENTAL DERMATOLOGY, COPENHAGEN, DK, vol. 7, no. 4, août 1998 (1998-08), pages 175-178, XP008001589 ISSN: 0906-6705
- PAWLIUK ET AL.: "Selection of retrovirally transduced hematopoietic cells using CD24 as a marker of gene transfer" BLOOD, vol. 84, no. 9, 1994, pages 2868-2877, XP008002532
- CARREAU M ET AL: "FUNCTIONAL RETROVIRAL VECTOR FOR GENE THERAPY OF XERODERMA PIGMENTOSUM GROUP D PATIENTS" HUMAN GENE THERAPY, vol. 6, no. 10, octobre 1995 (1995-10), pages 1307-1315, XP008001590 ISSN: 1043-0342

## Description

La présente invention a pour objet des procédés de préparation de lots homogènes de kératinocytes souches transformés avec une séquence exogène d'intérêt, ainsi que lesdits kératinocytes souches ainsi transformés, le tissu épidermique reconstitué à partir de ces kératinocytes, et leurs utilisations.

Les approches de transfert de gène dans les kératinocytes actuellement proposées sont confrontées aux problèmes résultant :
- soit de la sélection des cellules transduites en présence d'un agent pharmacologique léthal (type G418) ; ces techniques font toujours appel à un gène de résistance à un agent pharmacologique léthal (néomycine, hygromycine, blastdicidine, zéocine, etc..),
- soit de la non-sélection des cellules transduites

Il existe deux inconvénients majeurs à l'utilisation de gène de résistance à un agent pharmacologique léthal, et ce problème semble d'autant plus sensible dans le cas des kératinocytes épidermiques:
a-) le produit d'un gène d'origine exogène, à savoir du gène de résistance susmentionné, exprimé par les cellules transduites peut être reconnu comme du non-soi. Ceci engendre donc le problème de la réponse immunitaire si les cellules transduites sont destinées à la greffe sur un individu immunocompétent c'est à dire la forte probabilité d'un rejet de greffe à terme.
b-) l'épuisement au cours de la sélection du potentiel de croissance des cellules transduites, ce qui en soi limite les possibilités de prise, de maintien et de pérennité d'une éventuelle greffe de tissu généré à partir de ces cellules génétiquement modifiées.

La transduction des kératinocytes en absence de sélection permet de contourner les problèmes exposés ci-dessus : réponse immunitaire et épuisement du potentiel de croissance au cours de la sélection.

L'absence de sélection s'accompagne toutefois des problèmes :
- de l'homogénéité de la population des cellules transduites,
- de l'absence de sélection sur le critère des capacités de prolifération des cellules transduites.

Ces deux problèmes sont incompatibles avec des perspectives de thérapie cellulaire et tissulaire faisant appel aux kératinocytes, en particulier aux kératinocytes issus de patients atteints de Xeroderma pigmentosum (XP). Une population kératinocytaire transduite en absence de sélection contiendra nécessairement un faible % de cellules non-transduites, c'est à dire dont la sensibilité aux UV persistera. La persistance de ces cellules non-transduites dans la culture, dans un épiderme reconstruit et éventuellement dans un greffon, se traduira par la non-correction du phénotype de prédisposition aux carcinomes cutanés et donc de la réimplantation de cellules potentiellement tumorales.

D'autre part en absence de sélection, aucun avantage de croissance n'est donné aux cellules transduites par rapport aux cellules non-transduites. Une seule division est en effet nécessaire et suffisante pour l'infection rétrovirale. La pérennité d'un greffon réalisé à partir de cellules transduites en absence de sélection pourrait donc être inférieure à celle d'un greffon réalisé à partir de cellules sélectionnées sur leur capacité de prolifération comme cela est proposé dans le cadre de la présente invention.

Depuis plusieurs années les Inventeurs ont démontré que le phénotype de sensibilité aux UV des fibroblastes de XP en culture de patients atteints de xeroderma pigmentosum peut être reversé par transfert du gène de complémentation adéquat (Carreau et al., Cancinogenesis (1994), 15, 1493-1498; Quilliet et al., Mutation Res. (1996), 364, 161-169; Zheng et al., J. Dermatol. (1998), 18, 90-97). Plus précisément, ces études ont démontré que la réintroduction d'un gène XP restaure la capacité des cellules à réparer leur ADN au point de retrouver une survie aux UV normale. Toutefois, ces études ont
a-) été réalisées sur des fibroblastes de derme, cellules n'étant qu'exceptionnellement (< à 1 %) à l'origine de cancers cutanés, y compris chez les patients XP;
b-) utilisé des vecteurs rétroviraux équipés du gène de résistance à la néomycine et donc non utilisables pour des perspectives de réversion des kératinocytes épidermiques de XP.

L'invention a pour but de sélectionner à homogénéité des cellules épidermiques humaines (kératinocytes) en culture dans lesquelles un gène exogène a été introduit. Ces cellules génétiquement modifiées pourront être soit utilisées à des fins expérimentales, soit greffées chez le sujet donneur après reconstitution du tissu épidermique *in vitro.* L'avantage majeur de l'invention par rapport au système de sélection déjà connu réside sur le caractère naturel du marqueur de sélection et donc la possibilité de réimplanter les cellules transduites sans rejet par le malade d'une substance exogène inconnue.

L'invention découle de la mise en évidence par les Inventeurs que dans l'épiderme seuls les kératinocytes post-mitotiques expriment naturellement le marqueur CD24 à leur surface, tandis que les kératinocytes souches n'expriment pas ce marqueur CD24.

Il faut noter que le CD24 est naturellement exprimé dans l'épiderme. Ceci constitue toute la différence entre l'utilisation du CD24 versus des marqueurs de sélection utilisés classiquement (gènes de résistance à un agent pharmacologique, notamment à un antibiotique tel que le G418 dont la résistance est portée par le gène de résistance bactérien *néo*), car, contrairement au CD24, ceux-ci ne sont pas naturellement exprimés dans les kératinocytes épidermiques ou dans l'épiderme et donc sujet à réaction immunitaire, voire au rejet de la greffe. Plus précisément, comme mentionné ci-dessus, le CD24 est naturellement exprimé dans les couches suprabasales postmitotiques (qui ont perdu la capacité de se diviser) de l'épiderme humain, mais n'est pas exprimé à un niveau détectable dans les cellules de la couche basale de ce tissu. Le principe de l'invention est basé sur cette spécificité d'expression du CD24 à l'intérieur du tissu et la prise en compte des propriétés de prolifération différentielle des compartiments basal et suprabasal des cellules épidermiques. En effet, les cellules basales de l'épiderme sont les seules capables de se multiplier *in vivo* et sont à l'origine du renouvellement permanent du tissu. De plus, ces cellules peuvent être cultivées *in vitro.* La capacité multiplicative des cellules basales épidermiques en culture les rend permissives aux rétrovirus et en particulier aux rétrovirus dérivés du virus murin leucémogène de Moloney (MoMLV) produit par des cellules d'empaquetage productrices de particules infectieuses à enveloppe amphotrope.

Ainsi l'invention vise à contourner les limitations susmentionnées dans la mesure où elle est basée sur l'utilisation d'un marqueur de la surface cellulaire, le CD24 qui est :
a-) naturel dans l'épiderme,
b-) dont l'expression n'interfère pas avec la capacité proliférative des kératinocytes épidermiques,
c-) avantageusement sous contrôle du promoteur transcriptionnel contenu dans le LTR 5' de l'ADN proviral, c'est à dire dont l'expression s'éteindra environ 4 semaines après greffe d'un épiderme reconstruit à partir des cellules transduites (Choate et al., Hum. Gen. Ther. (1997), 8, 895-901).

Le principe de l'invention est basé sur une nouvelle méthode de sélection des kératinocytes exprimant le gène exogène d'intérêt par le biais de la coexpression de ce dernier, avec un marqueur de surface, le CD24. Les deux gènes (gène d'intérêt ou exogène, et CD24) sont présents dans la même construction d'ADN proviral. Le contrôle transcriptionnel de chacun de ces gènes est assuré par deux promoteurs indépendants (par exemple LTR 5' pour le gène CD24 , et un promoteur interne « physiologique » comme par exemple le promoteur du gène de la kératine humaine K5 pour le contrôle transcriptionnel du gène d'intérêt).

Le principe de sélection est donc applicable aux kératinocytes en cultures dans le cadre de la présente invention. Dans une culture kératinocytaire dérivée de l'épiderme humain, on trouvera schématiquement deux populations cellulaires. Une population capable de se multiplier et n'exprimant pas le CD24. Cette population est donc infectable par les particules virales amphotropes; une population ne se multipliant pas, engagée dans le processus irréversible de la différenciation et exprimant le CD24. Lors de l'infection d'une culture kératinocytaire hétérogène en terme de prolifération et d'expression du CD24, la transduction du gène du CD24 dans les cellules en division conduira à l'expression ectopique de la protéine correspondante (le CD24). A l'issu de l'infection rétrovirale, l'ensemble de la population cellulaire exprimant le CD24, soit de façon naturelle (cellules différenciées, postmitotiques ou en voie de l'être), soit de façon exogène (cellules transduites) pourra être sélectionnée par tri cellulaire à l'aide d'un anticorps spécifique du CD24. L'avantage de cette sélection est de ne pas perturber le métabolisme cellulaire de la culture infectée et donc de conserver toutes ses capacités biologiques qui seront utiles pour la suite des expériences. Parmi les cellules ainsi sélectionnées et remises en culture, seule celles exprimant le CD24 de façon exogène, c'est à dire douées d'une capacité de prolifération, seront conservées dans les boites de cultures. Qu'elles soient transduites ou non, toutes les cellules sélectionnées pour l'expression du CD24, et possédant une capacité de prolifération faible à nulle, seront éliminées au cours de la culture après quelques passages *in vitro.* Les cellules possédant des capacités prolifératives élevées (cellules souches) et CD24-positives (i.e. transduites) pourront être utilisées pour reconstruire un tissu épidermique génétiquement modifié.

Ainsi, comme cela a été mentionné ci-dessus, l'objet de la présente invention est de contourner les limitations susmentionnées dans la mesure où le CD24, à savoir le marqueur de sélection utilisé, est,
a)- exprimé naturellement dans l'épiderme (problème de la tolérance immunitaire),
b)- son expression à la surface des cellules transduites permet la sélection des kératinocytes clonogéniques conduisant à une population homogène.
c-) la méthode de sélection permet l'enrichissement en cellules progénitrices de l'épiderme ce qui est en faveur de la pérennité d'un éventuel greffon d'épiderme à partir de cellules génétiquement corrigées.

Les avantages de la méthode de sélection de la présente invention sont :
- l'immunotolérance par un organisme hôte d'un greffon réalisé à partir des cellules génétiquement modifiées,
- la possibilité de réaliser une sélection totale, mais sans épuisement des cultures par un agent pharmacologique ; cette stratégie permet d'améliorer la pérennité d'un épiderme de culture réalisé à partir des cellules génétiquement modifiées,
- le tri des cellules transduites est très facile et très efficace, faisable à l'échelle du laboratoire et sans équipement lourd spécifique ; le cas échéant, le seul matériel spécifique nécessaire est un anticorps secondaire,
- la construction permet d'exploiter le fait de la perte d'expression du gène CD24 peu de temps après greffe d'un épiderme réalisé à partir des cellules génétiquement modifiée. En effet, dans ce contexte, l'activite transcriptionnelle du LTR 5'par exemple, est perdue 3 à 4 semaines après greffe (Choate et al., Hom. Gen. Ther. (1997), 8, 895-901). Cette perte d'activité du LTR 5' conduit à la perte de l'expression exogène du marqueur de sélection dans la couche basale de l'épiderme reconstruit greffé. Ceci permet de minimiser les éventuels risques de perturber la physiologie du tissu épidermique pouvant être inhérents à l'expression du CD24.

Parmi les applications possibles de la présente invention, on peut citer notamment, dans le domaine de la génétique, l'étude des effets de la surexpression de gènes pouvant être impliqués dans l'homéostase du tissu épidermique ou de leur version mutée.

Une application possible est la construction d'épiderme humain exprimant une copie mutée d'un gène de complémentation XP aboutissant à l'hyperphotosensibilité du tissu, ou la possibilité d'incorporer des mélanocytes dans le tissu reconstruit afin d'évaluer le rôle de ces cellules dans un contexte photosensible lié à une déficience en réparation de l'ADN.

La présente invention a pour objet l'utilisation du gène marqueur CD24 de mammifères, tel que le gène CD24 humain, pour la transformation de kératinocytes souches dans le cadre de la mise en oeuvre de procédés de préparation de lots homogènes de cellules souches épidermiques transformées de manière à contenir une séquence nucléotidique exogène d'intérêt.

Avantageusement les gènes marqueurs CD24 de mammifères, et plus particulièrement le gène CD24 humain, utilisés dans le cadre de la présente invention, sont ceux décrits notamment dans l'article suivant : Kay R, Rosten PM, Humphries RK. CD24, a signal transducer modulating B cell activation response, is a very short peptide with a glycosyl phosphatidylinositol membrane anchor. J. Immunol. (1991), 147, 1412-1416.

L'invention a également pour objet un procédé de préparation de lots homogènes de cellules souches épidermiques transformées de manière à contenir une séquence nucléotidique exogène d'intérêt, caractérisé en ce qu'il comprend les étapes suivantes :
- la transformation de cellules de l'épiderme en culture comprenant des kératinocytes souches et des kératinocytes post-mitotiques de la couche suprabasale de l'épiderme sécrétant naturellement la protéine CD24 à l'aide d'un vecteur approprié dont le génome comprend une séquence recombinante contenant une séquence nucléotidique exogène d'intérêt et le gène CD24 en tant que marqueur de sélection, ce qui conduit à l'obtention d'un lot hétérogène de cellules, comprenant les kératinocytes souches et post-mitotiques susmentionnés transformés ou non avec la séquence recombinante du vecteur susmentionné,
- sélection des cellules transformées lors de l'étape précédente et exprimant à leur surface la protéine CD24, à l'aide d'un réactif reconnaissant spécifiquement cette protéine, tel que des anticorps spécifiques de CD24, ce qui conduit à l'obtention d'un lot hétérogène de cellules comprenant des kératinocytes souches et post-mitotiques susmentionnées transformés avec la séquence recombinante du vecteur susmentionné, et le cas échéant, de kératinocytes post-mitotiques susmentionnés non transformés avec ladite séquence recombinante,
- la mise en culture des cellules sélectionnées lors de l'étape précédente pendant un temps suffisant permettant d'assurer l'élimination des kératinocytes post-mitotiques susmentionnés, transformés ou non avec ladite séquence recombinante, susceptibles d'être présents lors de l'étape précédente de transformation et étant incapables de se diviser et donc d'être maintenus dans le milieu de culture, ce qui conduit à l'obtention d'un lot homogène de kératinocytes souches CD24⁺ contenant ladite séquence recombinante, capables de se diviser et d'être maintenus en culture.

L'invention a plus particulièrement pour objet un procédé tel que décrit ci-dessus, caractérisé en ce que la séquence nucléotidique exogène d'intérêt est choisie parmi :
- les séquences nucléotidiques susceptibles d'être utilisées en thérapie génique, lesdites séquences étant choisies notamment parmi les gènes de réparation de l'ADN impliqués dans les syndromes suivants : le xeroderma pigmentosum, à savoir les gènes XPA, XPB, XPC, XPD, XPE, XPG, XPF, la trichotiodystrophie, à savoir les gènes CSA et CSB, le syndrome de Cockayne, à savoir le gène LG-1, ou les gènes codant pour les facteurs intervenant dans la coagulation sanguine, tel que le gène codant pour le facteur VIII dans le cadre du traitement de l'hémophilie A, ou encore le gène codant pour l'insuline dans le cadre du traitement du diabète insuline-dépendant,
- les séquences nucléotidiques utilisables en tant que gènes traceurs pour suivre le devenir d'une cellule ou d'une population de cellules transduites exprimant ce gène marqueur, lesdites séquences étant choisies notamment parmi celles codant pour la GFP (Green Fluorescent Protein), la luciferase, ou pour la beta-galactosidase de *E. coli*,
- les séquences nucléotidiques comportant une mutation génétique dominante à effet phénotypique dont les effets sur la physiologie du tissu pourront être étudiés, lesdites séquences étant choisies notamment parmi celles susmentionnées correspondant aux gènes de réparation de l'ADN, et étant impliqués dans un ou plusieurs processus de réparation de l'ADN,
- les séquences nucléotidiques susceptibles de permettre le criblage de molécules d'intérêt thérapeutique sur l'épiderme, ou d'intérêt cosmétologique, lesdites séquences étant choisies notamment parmi les séquences de contrôle de la transcription de gènes dont l'expression est modulée après irradiation UV, notamment parmi les séquences suivantes :

- les séquences promotrices du gène c-Jun, de la collagénase ;
- les séquences, éventuellement synthétiques, comprenant les éléments de réponse de réponse aux UV (ultraviolet response element, URE) TGACAACA ;
- les séquences de fixation de la protéine P53, dont l'expression est stabilisée par les ultraviolets, notamment :
   . les séquences contenues dans le gène MDM2,
   . les séquences contenues dans le gène p21/cip1/WAF,
- les séquences, éventuellement synthétiques, comprenant un ou plusieurs éléments de réponse à P53.

Ces séquences sont avantageusement placées en amont d'un gène traceur (tel que défini ci-dessus). Les kératinocytes transduits et triés selon le procédé décrit ci-dessus permettent de reconstruire du tissu épidermique *in vitro* (en trois dimensions) selon la méthode décrite ci-après. L'irradiation ultraviolette de ces épidermes reconstruits *in vitro* entraînera une modulation (négative ou positive) de l'expression du gène traceur; cela permet d'évaluer qualitativement et quantitativement l'effet *in situ* de produits tels que :
- les substances cosmétiques (notamment les filtres solaires),
- les médicaments présentant une influence négative ou positive sur la réparation des lésions induites par les UV dans l'ADN, et de fait l'expression du gène traceur.

Avantageusement, le vecteur utilisé pour la transformation de cellules de l'épiderme en culture dans le cadre du procédé tel que défini ci-dessus; est choisi parmi les rétrovirus tels que les rétrovirus dérivés du virus murin leucémogène de Moloney, ou les vecteurs de type lentivirus, dérivés de HIV (ceux-ci présentent l'intérêt de permettre l'infection des cellules en absence de division).

L'invention a plus particulièrement pour objet tout procédé tel que défini ci-dessus, caractérisé en ce que l'étape de sélection des cellules transformées à l'aide d'anticorps spécifiques de CD24 est effectuée à l'aide d'anticorps reconnaissant spécifiquement les anticorps CD24 et couplés à des billes magnétiques, les cellules marquées CD24+ étant séparées des cellules non marquées à l'aide d'un aimant.

L'invention a également pour objet tout procédé tel que défini ci-dessus, caractérisé en ce que l'étape de mise en culture des cellules sélectionnées, à l'aide d'un réactif reconnaissant spécifiquement la protéine CD24, notamment un anticorps anti-CD 24, afin d'éliminer les kératinocytes post-mitotiques est effectuée de la manière suivante : les kératinocytes sélectionnés à l'aide de l'anticorps anti-CD24 sont ensemencés selon la méthode de Rheinwald JG, and Green H (Serial cultivation of strains of human epidermal keratinocytes : the formation of keratinizing colonies from single cells. Cell. 1975, 6, 331-43), à savoir sur une couche de fibroblastes nourriciers murins préalablement irradiés létalement aux rayons gamma ; dans ces conditions, les cellules post-mitotiques sélectionnées ne s'attachent pas au support ; des cellules sub- ou pré-postmitotiques peuvent s'attacher, mais forment des colonies dites abortives donnant naissance à des cellules post-mitotiques qui sont éliminées après un passage en culture. Ainsi, les cellules post-mitotiques sont éliminées soit immédiatement après ensemencement (cellules ne s'attachant pas au support), soit après un passage en culture.

L'invention concerne également les kératinocytes souches de mammifères, et plus particulièrement humains, transformés de manière à ce que leur génome contienne le gène marqueur CD24 de mammifères, et plus particulièrement le gène CD24 humain.

L'invention a plus particulièrement pour objet les kératinocytes souches transformés susmentionnés, caractérisés en ce que leur génome contient au moins une séquence nucléotidique exogène d'intérêt telle que définie ci-dessus.

L'invention a également pour objet des lots homogènes de kératinocytes souches transformés susmentionnés, et plus particulièrement de lots de kératinocytes souches transformés dont le taux d'homogénéité est avantageusement supérieur à environ 99%, à savoir dont la proportion de kératinocytes souches non transformés et/ou de kératinocytes post-mitotiques transformés ou non est nulle ou inférieur à environ 1 %.

L'invention concerne également les kératinocytes souches transformés et les lots homogènes de ces derniers susmentionnés, tels qu'obtenus par mise en oeuvre d'un procédé tel que défini ci-dessus.

L'invention a également pour objet du tissu épidermique reconstitué à partir de kératinocytes souches transformés ou de lots homogènes de ces derniers tels que définis ci-dessus.

L'invention concerne également les greffons de tissu épidermique reconstitué tel que susmentionné.

L'invention a plus particulièrement pour objet l'utilisation de tissu épidermique tel que défini ci-dessus, pour la préparation de greffons susmentionnés, destinés au traitement de pathologies liées à un désordre génétique.

Les techniques utilisées pour la préparation de tissu épidermique reconstitué selon l'invention sont décrites notamment dans les articles suivants : Gallico et al., N. Engl. Med. 311, 448-451, 1984). Bell et al., 1979 Proc Natl Acad Sci USA 76, 274-1278, et Asselineau et al., 1984, Exp. Cell. Res, 159, 536-539.

L'invention a également pour objet l'utilisation de kératinocytes souches transformés ou de lots homogènes de ces derniers tels que définis ci-dessus, ou de tissu épidermique reconstitué susmentionné, pour la mise en oeuvre de :
- procédés de suivis *in vitro* du devenir d'une cellule ou d'une population de cellules transduites exprimant des séquences nucléotidiques exogènes d'intérêt en tant que gènes marqueurs,
- de procédés d'étude *in vitro* des effets sur la physiologie du tissu épidermique de séquences nucléotidiques exogènes d'intérêt correspondant à des séquences comportant une mutation génétique dominante à effet phénotypique,
- de procédés de criblage *in vitro* de molécules d'intérêt thérapeutique sur l'épiderme, ou d'intérêt cosmétologique, susceptibles d'avoir un effet directement sur la séquence nucléotidique exogène d'intérêt, ou indirectement sur l'état physiologique cellulaire induit par ladite séquence nucléotidique exogène d'intérêt, ces derniers procédés comprenant une étape de mise en présence desdites molécules d'intérêt avec les kératinocytes souches transformés ou le tissu épidermique transformé susmentionné.

L'invention sera davantage illustrée à l'aide de la description détaillée qui suit de l'obtention de lots homogènes de kératinocytes souches transformés selon l'invention.

### 1. Construction de nouveaux ADN proviraux recombinants

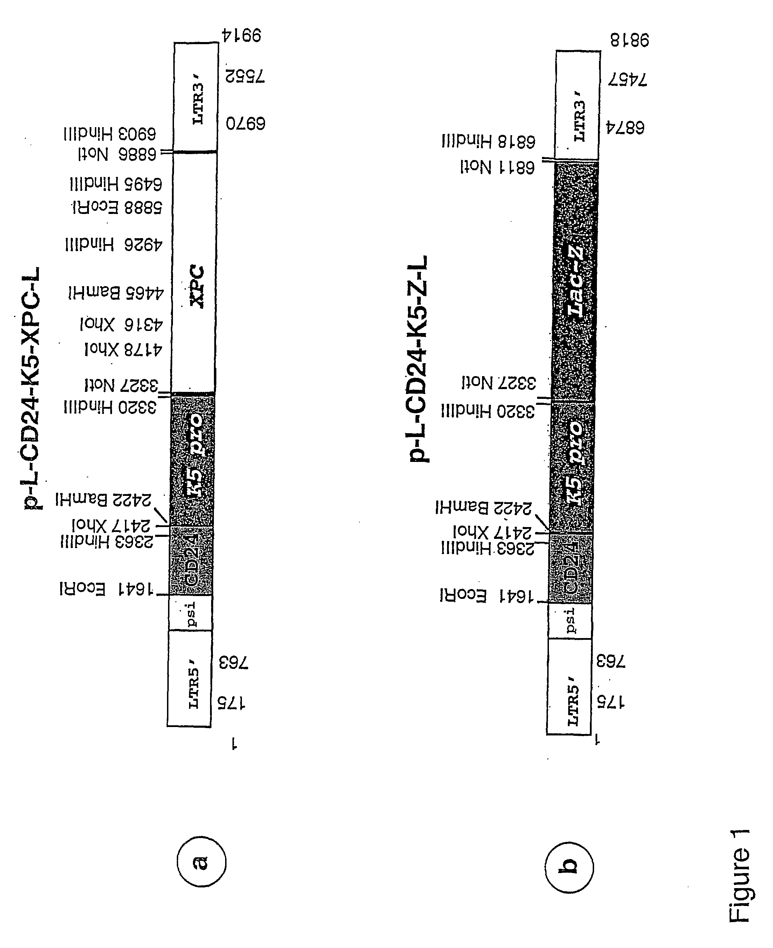

Nous avons construit par génie génétique de nouveaux ADN proviraux recombinants, dérivés de l'ADN proviral de MoMLV (figure 1a, 1b). Dans ces constructions, le CD24 est exprimé à partir de son ADN complémentaire dont la transcription est contrôlée en amont par le promoteur présent dans le LTR 5' de l'ADN proviral de MoMLV. D'autre part, un promoteur interne, le promoteur du gène de la kératine K5 humaine (promoteur K5), est placé en amont du gène de complémentation *XPC*. Le promoteur K5 permet de cibler l'expression du gène XPC dans les cellules basales, prolifératives de l'épiderme, car celles-ci sont à l'origine des carcinomes cutanés (épithélioma baso- et spino cellulaires). Le gène *XPC* est destiné à complémenter la déficience en réparation des dommages induits par le ultraviolets B (290-320 nm) qui est responsable de la photosensibilité et la prédisposition aux carcinomes cutanés des kératinocytes de patients atteints de xeroderma pigmentosum (XP) appartenant au groupe de complémentation C. Le gène *XPC* a été choisi :
a- ) en raison de la fréquence de ce groupe de complémentation dans notre région du globe (> 50 %)
b-) en raison du tableau clinique des patients XP-C qui sont relativement épargnés d'autres problèmes que la déficience en réparation de l'ADN, comme par exemple les dégénérescences neurologiques. En effet le développement staturopondéral et neurologique est normal chez ces malades.

### 2. Applications dérivées de la construction

En utilisant, le « squelette » de la construction montrée à la figure 1a, tout autre gène XP, jouant un rôle distinct de celui de XPC dans une étape de réparation de l'ADN et étant impliqué dans la pathologie XP, peut être inséré à la place de XPC pour assurer la complémentation dans les kératinocytes provenant des patients appartenant aux autres groupes de complémentation de XP (i.e. XP-A, B, D, E, F, G).

De même, tout autre gène d'intérêt (gène traceur, sous contrôle d'un promoteur d'intérêt, par exemple le gène bactérien lac Z), peut être inséré à la place du gène *XPC* comme illustré à la figure 1b.

N'importe quel autre gène d'intérêt, sauvage ou porteur d'une mutation inactivatrice de sa fonction originale, peut aussi être inséré à la place du gène XPC illustré à la figure 1a, afin d'étudier les effets, soit de la surexpression de ce gène, soit d'une mutation génétique donnée, déterminée à des fin expérimentales (analyse des relations structure fonction du gène), ou identifiée à partir d'échantillons humain (tumeur). Ce dernier exemple d'application permet de fabriquer des cellules mutantes et de construire, à partir d'une même souche de kératinocytes sauvages différentes souches de kératinocytes de même fond génétique mais présentant des mutations différentes sur le gène d'intérêt. Si les mutations concernent un gène de réparation de l'ADN, on peut par exemple mesurer l'effet de ces mutations sur la capacité des cellules à réparer les lésions UV-induites dans l'ADN et, le cas échéant, pronostiquer la prédisposition aux carcinomes cutanés des kératinocytes porteurs de la mutation.

N'importe quel autre promoteur d'intérêt, peut d'autre part être inséré à la place du promoteur du gène de la kératine K5 afin de contrôler en aval la transcription de tout gène d'intérêt : gène de complémentation, gène traceur, gène reporter, ou gène muté.

### 3. Procédé de transformation des kératinocytes et de sélection des kératinocytes souches transformés

Les kératinocytes ensemencés fraîchement (24 heures avant infection) sont incubées pendant 16-18 heures en présence de surnageant infectieux (contenant les particules virales et 5 µg/ml de polybrène, hexadimetrine bromide). Le surnageant infectieux à été obtenu au préalable par incubation des cellules productrices des particules virales une nuit en milieu « SFM, (GIBCO Bethesda Research Laboratories, USA), 0.1 mM CaCl2, sans autre nutriment.

Après infection les kératinocytes sont dissociées par traitement enzymatique à la trypsine, resuspendues à raison de 450 000 cellules/ml de milieu de culture des kératinocytes contenant 10 % de sérum de veau foetal décomplémenté (CFAD-DBS). Les cellules sont incubées à 4 °C pendant 1,5 heures en présence de 22 µg/10 ⁶ cellules d'anticorps primaire anti-CD24 (clone ALB9, Immunotech, Luminy France). Les cellules sont ensuite lavées 2 fois 10 minutes dans du CFAD-DBS puis incubées en présence d'un excès (x44) d'anticorps secondaire couplé à des billes métalliques (810 ⁷, M-450, DYNAL) préalablement lavées en tampon phosphate (PBS) contenant 0,1 % d'albumine bovine sérique (BSA). Les cellules complexées avec l'anticorps couplé aux billes métalliques sont séparées des autres cellules à l'aide d'un aimant. Cette étape est répétée 2 fois. Les cellules complexées ainsi séparées constituent la fraction CD24 positive. Les cellules non-complexées constituent la fraction CD24-négative.

Les cellules CD24-positives sont ensuite dissociées du complexe anticorps/billes métalliques par une incubation de 45 minutes en présence d'un troisième anticorps « dissociateur » (Detachbead CD19, M-450, Pan-B DYNAL).Les cellules ainsi dissociées sont ensuite séparées du complexe anticorps/billes métalliques, comptées puis ensemencées pour analyse ultérieure : mesure de l'expression du gène d'intérêt, fonctionnalité du produit du gène d'intérêt, capacité à reconstituer un épithélium de culture et un épiderme en trois dimensions.

Certaines méthodes de tri cellulaire, à l'aide de trieur de cellules permettent de fixer le seuil de pureté des cellules positives jusqu'à une pureté > 99.8 %.

### 4. Procédés de préparation d'épiderme reconstitué à partir des kératinocytes souches transformés

Plusieurs méthodes sont applicables :
- simple épithélium de culture cultivé à partir des cellules transduites et sélectionnées ensemencées puis portées à confluence. L'épithélium de culture est détaché du support plastique après traitement par l'enzyme « dispose II» (12.5 mg /ml) et appliqué sur le lit de greffe. Cette technique classique est utilisée pour les autogreffes dans le cadre du traitement des grands brûlés (Gallico et al., N. Engl. Med. 311, 448-451).
- Une autre technique utilisable est celle basée sur la fabrication décrite initialement par Bell et al., 1979 Proc Natl Acad Sci USA 76, 274-1278. et améliorée par Asselineau et al., 1984, Exp. Cell. Res, 159, 536-539.

### Schéma des nouveaux vecteurs

**Figure 1:** Constructions rétrovirales. CD24: CD24 cDNA; XPC et lac Z: cDNAs des gènes d'intérêt. K5 pro: 905 bp région promotrice de la transcription du gène humain de la kératine K5. LTR 5', LTR 3': « long terminal region » de l'ADN proviral de MoMLV. Le LTR 5' permet la transcription du cDNA CD24. Les ADN proviraux recombinants sont dérivés du vecteur LXSN (Clontech, accession #m28248).

## Revendications

1. Utilisation du gène marqueur CD24 de mammifères, tel que le gène CD24 humain, pour la transformation de kératinocytes souches dans le cadre de la mise en oeuvre de procédés de préparation de lots homogènes de cellules souches épidermiques transformées de manière à contenir une séquence nucléotidique exogène d'intérêt.

2. Procédé de préparation de lots homogènes de cellules souches épidermiques transformées de manière à contenir une séquence nucléotidique exogène d'intérêt, **caractérisé en ce qu'**il comprend les étapes suivantes :
- la transformation de cellules de l'épiderme en culture comprenant des kératinocytes souches et des kératinocytes post-mitotiques de la couche suprabasale de l'épiderme sécrétant naturellement la protéine CD24 à l'aide d'un vecteur approprié dont le génome comprend une séquence recombinante contenant une séquence nucléotidique exogène d'intérêt et le gène CD24 en tant que marqueur de sélection, ce qui conduit à l'obtention d'un lot hétérogène de cellules, comprenant les kératinocytes souches et post-mitotiques susmentionnés transformés ou non avec la séquence recombinante du vecteur susmentionné,
- sélection des cellules transformées lors de l'étape précédente et exprimant à leur surface la protéine CD24, à l'aide d'un réactif reconnaissant spécifiquement cette protéine, tel que des anticorps spécifiques de CD24, ce qui conduit à l'obtention d'un lot hétérogène de cellules comprenant des kératinocytes souches et post-mitotiques susmentionnées transformés avec la séquence recombinante du vecteur susmentionné, et le cas échéant, de kératinocytes post-mitotiques susmentionnés non transformés avec ladite séquence recombinante,
- la mise en culture des cellules sélectionnées lors de l'étape précédente pendant un temps suffisant permettant d'assurer l'élimination des kératinocytes post-mitotiques susmentionnés, transformés ou non avec ladite séquence recombinante, susceptibles d'être présents lors de l'étape précédente de transformation et étant incapables de se diviser et donc d'être maintenus dans le milieu de culture, ce qui conduit à l'obtention d'un lot homogène de kératinocytes souches CD24⁺ contenant ladite séquence recombinante, capables de se diviser et d'être maintenus en culture.

3. Procédé selon la revendication 2, **caractérisé en ce que** la séquence nucléotidique exogène d'intérêt est choisie parmi :
- les séquences nucléotidiques susceptibles d'être utilisées en thérapie génique,
- les séquences nucléotidiques utilisables en tant que gènes traceurs pour suivre le devenir d'une cellule ou d'une population de cellules transduites exprimant ce gène marqueur,
- les séquences nucléotidiques comportant une mutation génétique dominante à effet phénotypique dont les effets sur la physiologie du tissu pourront être étudiés,
- les séquences nucléotidiques susceptibles de permettre le criblage de molécules d'intérêt thérapeutique sur l'épiderme, ou d'intérêt cosmétologique.

4. Procédé selon la revendication 2 ou 3, **caractérisé en ce que** l'étape de sélection des cellules transformées à l'aide d'anticorps spécifiques. de CD24 est effectuée à l'aide d'anticorps reconnaissant spécifiquement les anticorps CD24 et couplés à des billes magnétiques, les cellules marquées CD24+ étant séparées des cellules non marquées à l'aide d'un aimant.

5. Kératinocytes souches de mammifères, et plus particulièrement humains, transformés de manière à ce que leur génome contienne le gène marqueur CD24 de mammifères, et plus particulièrement le gène CD24 humain.

6. Kératinocytes souches transformés selon la revendication 5, **caractérisé en ce que** leur génome contient au moins une séquence nucléotidique exogène d'intérêt telle que définie dans la revendication 3.

7. Lot homogène de kératinocytes souches transformés selon la revendication 5 ou 6, dont le taux d'homogénéité est avantageusement supérieur à environ 99%, à savoir dont la proportion de kératinocytes souches non transformés et/ou de kératinocytes post-mitotiques transformés ou non est nulle ou inférieur à environ 1%.

8. Kératinocytes souches transformés et lots homogènes de ces derniers selon l'une des revendications 5 à 7, tels qu'obtenus par mise en oeuvre d'un procédé selon l'une des revendications 2 à 4.

9. Tissu épidermique reconstitué à partir de kératinocytes souches transformés ou de lots homogènes de ces derniers selon l'une des revendications 5 à 8.

10. Greffon de tissu épidermique reconstitué selon la revendication 9.

11. Utilisation de tissu épidermique reconstitué selon la revendication 9, pour la préparation de greffons selon la revendication 10, destiné au traitement de pathologies liées à un désordre génétique.

12. Utilisation de kératinocytes souches transformés ou de lots homogènes de ces derniers selon l'une des revendications 5 à 8, ou de tissu épidermique reconstitué selon la revendication 9, pour la mise en oeuvre de :
- procédés de suivis *in vitro* du devenir d'une cellule ou d'une population de cellules transduites exprimant des séquences nucléotidiques exogènes d'intérêt en tant que gènes marqueurs,
- de procédés d'étude *in vitro* des effets sur la physiologie du tissu épidermique de séquences nucléotidiques exogènes d'intérêt correspondant à des séquences comportant une mutation génétique dominante à effet phénotypique,
- de procédés de criblage *in vitro* de molécules d'intérêt thérapeutique sur l'épiderme, ou d'intérêt cosmétologique, susceptibles d'avoir un effet directement sur la séquence nucléotidique exogène d'intérêt, ou indirectement sur l'état physiologique cellulaire induit par ladite séquence nucléotidique exogène d'intérêt, ces derniers procédés comprenant une étape de mise en présence desdites molécules d'intérêt avec, les kératinocytes souches transformés ou le tissu épidermique transformé susmentionné.

## Claims

1. Use of the CD24 marker gene of mammals, such as the human CD24 gene, for the transformation of stem keratinocytes in the implementation of processes for preparing homogenous batches of epidermal stem cells transformed in order to contain an exogenous nucleotide sequence of interest.

2. Process for preparing homogenous batches of epidermal stem cells transformed in order to contain an exogenous nucleotide sequence of interest, **characterized in that** it comprises the following stages:
- the transformation of cells of the epidermis in culture comprising stem keratinocytes and post-mitotic keratinocytes of the suprabasal layer of the epidermis naturally secreting the CD24 protein using an appropriate vector the genome of which comprises a recombinant sequence containing an exogenous nucleotide sequence of interest and the CD24 gene as selection marker, which leads to the obtaining of a heterogeneous batch of cells, comprising the above-mentioned stem and post-mitotic keratinocytes transformed or not transformed with the recombinant sequence of the above-mentioned vector,
- selection of the cells transformed during the preceding stage and expressing at their surface the CD24 protein, using a reagent specifically recognizing this protein, such as CD24-specific antibodies, which leads to the obtaining of a heterogeneous batch of cells comprising above-mentioned stem and post-mitotic keratinocytes transformed with the recombinant sequence of the above-mentioned vector, and if appropriate, of above-mentioned post-mitotic keratinocytes not transformed with said recombinant sequence,
- the culture of the cells selected during the preceding stage for a period of time sufficient to allow the elimination of the above-mentioned post-mitiotic keratinocytes, transformed or not transformed with said recombinant sequence, capable of being present during the preceding stage of transformation and being incapable of dividing and therefore of being maintained in the culture medium, which leads to the obtaining of a homogenous batch of CD24⁺ stem keratinocytes containing said recombinant sequence, capable of dividing and of being maintained in culture.

3. Process according to claim 2, **characterized in that** the exogenous nucleotide sequence of interest is chosen from:
- the nucleotide sequences capable of being used in gene therapy,
- the nucleotide sequences which can be used as tracer genes to follow the development of a cell or population of transduced cells expressing this marker gene,
- the nucleotide sequences comprising a dominant genetic mutation with phenotype effect, the effects of which on the physiology of the tissue can be studied,
- the nucleotide sequences which can allow the screening of molecules of therapeutic interest for the epidermis, or of cosmetological interest.

4. Process according to claim 2 or 3, **characterized in that** the stage of selection of the cells transformed using CD24-specific antibodies is carried out using antibodies specifically recognizing the CD24 antibodies and coupled to magnetic beads, the CD24+ marked cells being separated from the non-marked cells using a magnet.

5. Stem keratinocytes from mammals, and more particularly humans, transformed in order that their genome contains the CD24 marker gene of mammals, and more particularly the human CD24 gene.

6. Stem keratinocytes transformed according to claim 5, **characterized in that** their genome contains at least one exogenous nucleotide sequence of interest as defined in claim 3.

7. Homogenous batch of stem keratinocytes transformed according to claim 5 or 6, the homogeneity rate of which is advantageously above approximately 99%, namely the proportion of non-transformed stem keratinocytes and/or of transformed or non-transformed post-mitotic keratinocytes is nil or lower than approximately 1 %.

8. Transformed stem keratinocytes and homogenous batches of the latter according to one of claims 5 to 7, as obtained by implementation of a process according to one of claims 2 to 4.

9. Epidermal tissue reconstituted from transformed stem keratinocytes or homogenous batches of the latter according to one of claims 5 to 8.

10. Graft of epidermal tissue reconstituted according to claim 9.

11. Use of epidermal tissue reconstituted according to claim 9, for the preparation of grafts according to claim 10, intended for the treatment of pathologies linked to a genetic disorder.

12. Use of transformed stem keratinocytes or homogenous batches of the latter according to one of claims 5 to 8, or of epidermal tissue reconstituted according to claim 9, for the implementation of:
- processes for monitoring *the in vitro* development of a cell or population of transduced cells expressing exogenous nucleotide sequences which are of interest as marker genes,
- processes for studying *in vitro* the effects on the physiology of the epidermal tissue of exogenous nucleotide sequences of interest corresponding to sequences comprising a dominant genetic mutation with phenotype effect,
- processes for screening *in vitro* molecules of therapeutic interest for the epidermis, or of cosmetological interest, capable of having an effect directly on the exogenous nucleotide sequence of interest, or indirectly on the cell physiological state induced by said exogenous nucleotide sequence of interest, the latter processes comprising a stage of placing said molecules of interest in the presence of the transformed stem keratinocytes or the above-mentioned transformed epidermal tissue.

## Patentansprüche

1. Verwendung des Markergens CD24 von Säugetieren, wie des humanen CD24-Gens, für die Transformation von Stammkeratinozyten im Rahmen der Durchführung von Herstellungsverfahren für homogene Mengen von epidermalen Stammzellen, die derart transformiert sind, dass sie eine exogene Nukleotidsequenz von Interesse enthalten.

2. Verfahren zur Herstellung von homogenen Mengen von epidermalen Stammzellen, die derart transformiert sind, dass sie eine exogene Nukleotidsequenz von Interesse enthalten, **dadurch gekennzeichnet, dass** es die folgenden Schritte umfasst:
- die Transformation von Epidermiszellen, die Stammkeratinozyten sowie post-mitotische Keratinozyten der suprabasalen Epidermisschicht, die das Protein CD24 natürlich sezernieren, umfassen, welche Transformation in Kultur mit Hilfe eines geeigneten Vektors durchgeführt wird, dessen Genom eine rekombinante Sequenz enthält, die eine exogene Nukleotidsequenz von Interesse und das CD24-Gen als Selektionsmarker enthält, was zum Erhalt einer heterogenen Zellmenge führt, die die oben genannten Stammkeratinozyten und post-mitotischen Keratinozyten mit der rekombinanten Sequenz des obengenannten Vektors transformiert oder nicht transformiert enthält,
- die Selektion der nach dem obigen Schritt transformierten Zellen, die an ihrer Oberfläche das Protein CD24 exprimieren, mit Hilfe eines Reagens, das dieses Protein spezifisch erkennt, wie für CD24 spezifische Antikörper, was zum Erhalt einer heterogenen Zellmenge führt, die Stammkeratinozyten und post-mitotische Keratinozyten, die mit der rekombinanten Sequenz des obengenannten Vektors transformiert sind, und gegebenenfalls oben erwähnte post-mitotische Keratinozyten, die nicht mit der rekombinanten Sequenz transformiert sind, enthält,
- das Einbringen der nach dem obigen Schritt selektionierten Zellen in Kultur für eine Zeitdauer, die es erlaubt, die Eliminierung der obigen post-mitotische Keratinozyten sicherzustellen, die mit der genannten rekombinanten Sequenz transformiert wurden oder nicht, die während des vorhergehenden Transformationsschrittes anwesend sein können und nicht fähig sind, sich zu teilen, und daher nicht im Milieu der Kultur erhalten bleiben können, was zum Erhalt einer homogenen Menge von die genannte rekombinante Sequenz enthaltenden CD24⁺ Stammkeratinozyten führt, die sich teilen und in Kultur erhalten werden können.

3. Verfahren nach Anspruch 2, **dadurch gekennzeichnet, dass** die exogene Nukleotidsequenz von Interesse gewählt wird aus:
- Nukleotidsequenzen, die fähig sind für Gentherapie verwendet zu werden,
- Nukleotidsequenzen, die als Gentracer verwendet werden können, um die Entwicklung einer Zelle oder einer Population transduzierter Zellen, die diesen Genmarker exprimieren, zu verfolgen,
- Nukleotidsequenzen, die eine dominante genetische Mutation mit phenotypischer Wirkung tragen, deren Wirkungen auf die Physiologie des Gewebes studiert werden können,
- Nukleotidsequenzen, die fähig sind, das Auswählen von Molekülen mit hauttherapeutischem Interesse oder kosmetischem Interesse zu erlauben.

4. Verfahren nach Anspruch 2 oder 3, **dadurch gekennzeichnet, dass** der Schritt der Selektion der transformierter Zellen mit Hilfe von Antikörpern, die für CD24 spezifisch sind, mit Hilfe von Antikörpern bewirkt wird, die spezifisch die CD24 Antikörper erkennen und an Magnetkugeln gekoppelt sind, wobei die CD24⁺ markierten Zellen von den nicht markierten Zellen mit Hilfe eines Magneten getrennt werden.

5. Stammkeratinozyten von Säugetieren, und insbesondere vom Menschen, die derart transformiert sind, dass ihr Genom das CD24 Markergen von Säugetieren enthält, und insbesondere das humane CD24 Gen.

6. Stammkeratinozyten transformiert gemäß Anspruch 5, **dadurch gekennzeichnet, dass** ihr Genom zumindest eine exogene Nukleotidsequenz von Interesse wie in Anspruch 3 definiert enthält.

7. Homogene Menge an Stammkeratinozyten transformiert gemäß Anspruch 5 oder 6, deren Homogenitätsgrad vorzugsweise größer als etwa 99% ist, nämlich deren Anteil an nicht transformierten Stammkeratinozyten und/oder transformierten oder nicht transformierten post-mitotischen Keratinozyten null oder unter etwa 1% ist.

8. Transformierte Stammkeratinozyten und homogene Mengen der letzteren gemäß einem der Ansprüche 5 bis 7, wie jene, die durch die Durchführung eines Verfahrens gemäß einem der Ansprüche 2 bis 4 erhalten werden.

9. Epidermisgewebe hergestellt aus transformierten Stammkeratinozyten oder homogenen Mengen der letzteren gemäß einem der Ansprüche 5 bis 8.

10. Hautgewebetransplantat hergestellt gemäß Anspruch 9.

11. Verwendung des hergestellten Epidermisgewebes gemäß Anspruch 9 für die Herstellung von Transplantaten nach Anspruch 10 für die Behandlung von Pathologien, die mit genetischen Defekten verbunden sind.

12. Verwendung von transformierten Stammkeratinozyten oder homogenen Mengen der letzteren gemäß einem der Ansprüche 5 bis 8, oder von hergestelltem Epidermisgewebe nach Anspruch 9 zur Durchführung von:
- Verfahren *zum in vitro* Verfolgen der Entwicklung einer Zelle oder einer Population von transduzierten Zellen, die exogene Nukleotidsequenzen von Interesse als Genmarker exprimieren,
- Verfahren zum *in vitro* Untersuchen von Wirkungen auf die Physiologie von Hautgewebe mit exogenen Nukleotidesequenzen von Interesse, die Sequenzen entsprechen, die eine dominante genetische Mutation mit phenotypischer Wirkung tragen,
- Verfahren zum *in vitro* Auswählen von Molekülen von hauttherapeutischem oder kosmetischem Interesse, die fähig sind, direkt auf die exogene Nukleotidsequenz von Interesse oder indirekt auf den physiologischen Zellzustand zu wirken, der durch die exogene Nukleotidsequenz von Interesse induziert wird, wobei diese letzteren Verfahren einen Schritt enthalten, die Moleküle von Interesse mit den transformierten Stammkeratinozyten oder dem oben erwähnten transformierten Epidermisgewebe einzusetzen.
